# EUROPEAN PATENT SPECIFICATION

(11) **EP 3 493 213 B1**
(45) Date of publication and mention of the grant of the patent: **30.12.2020**
(21) Application number: 18156444.4
(22) Date of filing: 13.02.2018
(51) Int. Cl.: G16H 30/00, H04N 13/383, G06F 3/01, G06F 3/0484, G16H 30/40, G16H 40/60, G16H 50/20

(54) **METHOD, SYSTEM, COMPUTER PROGRAM PRODUCT AND COMPUTER-READABLE MEDIUM FOR SUPPORTING A VISUAL ANALYSIS OF A MEDICAL IMAGE DATA SET**
VERFAHREN, SYSTEM, COMPUTERPROGRAMMPRODUKT UND COMPUTERLESBARES MEDIUM ZUR UNTERSTÜTZUNG EINER VISUELLEN ANALYSE EINES MEDIZINISCHEN BILDDATENSATZES
PROCÉDÉ, SYSTÈME, PRODUIT-PROGRAMME D'ORDINATEUR ET SUPPORT LISIBLE PAR ORDINATEUR POUR PRENDRE EN CHARGE UNE ANALYSE VISUELLE D'UN ENSEMBLE DE DONNÉES D'IMAGES MÉDICALES

(43) Date of publication of application: 05.06.2019
(73) Proprietor: Siemens Healthcare GmbH, 91052 Erlangen (DE)
(72) Inventor: Blaha, Andreas, 96114 Hirschaid (DE)

(56) References cited:
- WO-A1-2017/033516
- JP-A- 2010 035 756
- US-A1- 2015 072 371
- US-A1- 2017 045 938
- US-B1- 8 020 993

## Description

The present invention deals with a method, a system, a computer program product and a computer-readable medium for supporting a visual analysis of a medical image data set.

The visualisation of medical image data sets, such as data sets being recorded via a computer tomography scanner or a magnetic resonance tomography scanner, is well known from the state of the art. Typically, the medical image data set is reconstructed, and as a result of such a reconstruction a visualized medical image data set is presented on a screen to a user, for example, to a radiologist. In particular, the visualized medical image data set comprises several cross sectional views that, for example, give insight into an examined organ, layer by layer.

For identifying abnormalities, the user has to visually examine the visualized medical image data sets, in particular layer by layer. Due to a huge amount of details that have to be considered in the examination, there is a chance that a region on the screen is not appropriately observed and/or viewed by the user and, in the worst case, that a relevant aspect for a further treatment of the patient might be overseen by the user. As a consequence, it is usually provided that the visualized medical image data sets have to be analysed a second time by another user. Thus, a probability for overseeing a crucial aspect on the visualized medical image data set is reduced. However, this procedure is both resource and time consuming.

According to JP 10 1 0035 756 it is well known to divide a diagnostic image into the prescribed number of areas and an image feature is calculated for each area. For example, when the diagnostic person observes the diagnostic image, the observation time in each area of the diagnostic image is detected using line-of sight detection.

US 2017 0 045 938 A1 concerns an image diagnosis assistance apparatus that determines an observed region in a tomographic image based on the detected gaze position and identifies the observed region in the three-dimensional medical image.

US 8,020,993 B1 describes a viewing verification system that allows a used to view an image by providing a viewer for the image, determining the space bounded by the foveal-vision of the user, verifying whether substatinally the entire meaningful portion of the image has been correlated to the space bounded by the foveal vision of the user.

WO 2017 033516 concerns a radiograph interpretation assistance device and a method which prevent areas of a radiograph to be interpreted from being overlooked by a radiologist without interfering with the radiologist's interpretation.

It is therefore an object of the present invention to provide a method and a system for further reducing the probability of missing a relevant aspect in a visualized medical image data set, in particular in a less resource and time consuming manner.

This object is achieved by the method according to claim 1 for supporting a visual analysis of a medical image data set, by the system according to claim 12, the computer program product according to claim 13, and by the computer-readable computer medium according to claim 14.

According to a first aspect of the present invention, a method for supporting a visual analysis of a medical image data set is provided, comprising:
- providing a visualized medical image data set;
- providing a device for eye-tracking for observing an eye orientation of a user during an analysis of the visualized medical image data set;
- assigning eye orientations of the user to regions of the visualized medical image date set for calibration;
- performing the analysis of the visualized medical image data set by the user;
- acquiring the eye orientation of the user during analysis by the device for eye-tracking;
- assigning a control value to a first region based on an intensity of acquired eye orientations directed to the first region and
- providing an information to the user when the control value is smaller and/or bigger than a threshold value for the first region.

Contrary to the state of the art, the user is informed or warned by the provided information when the first region has not been observed and/or viewed enough. Thereby, the decision for informing the user is based on results of analysing the eye orientation of the user, preferably by using an eye-tracking device. As a consequence, the probability for missing an abnormality in the visualized medical image data set is advantageously reduced, and possibly, a further re-examination of the visualized medical image data set is not necessary. Therefore, shorter treatment times and improved treatments are possible, leading to reduced overall cost for treating a patient.

The term "visualized medical image data set" preferably relates to a medical image that is reconstructed from a medical data set that, in turn, is recorded by a medical imaging device, such as a Computer Tomography (CT) scanner or a Magnetic Resonance Tomography (MRT) scanner. In general, the visualized medical image data set is presented on a screen or display of a computer, a tablet and/or smartphone such that the user can analyse the visualized medical image data set. In particular, the visualized medical image data set comprises several cross-sectional views that have to be analysed by the user one after the other. For analysing the visualized medical image data set, the user looks at the visualized medical image data set. Preferably, a processor is provided, wherein the processor is configured for
- providing a visualized medical image data set and/or
- assigning eye orientations of the user to regions of the visualized medical image date set for calibration and/or
- assigning a control value to a first region based on an intensity of acquired eye orientations directed to the first region and/or
- providing an information to the user when the control value is smaller and/or bigger than a threshold value for the first region.

Thereby, it is conceivable that the processor is part of a network or is part of a working station of the scanner. Preferably, the processor is configured for performing the reconstruction and/or the providing the information. In particular, the results of the reconstruction, i.e., the visualized medical image data set and/or the information, are visualized on a screen or display, such as on a screen or display of a workstation, smartphone and/or tablet. A workstation might be a (personal) computer, a virtual running machine on host hardware, a microcontroller, or an integrated circuit. As an alternative, the workstation can be a real or a virtual group of computers. Preferably, the workstation comprises a calculation unit and a memory unit. A calculation unit can comprise hardware elements and software elements, for example, the processor, in particular a microprocessor, or a field programmable gate array. A memory unit can be embodied as a non-permanent main memory (e.g. random access memory) or a permanent mass storage (e.g. a hard disk, USB stick, SD card, solid state disk). Preferably, the workstation can be part of the medical imaging device. It is also thinkable that at least one of the steps of the method is performed on a server or at the cloud.

In particular, the visualized medical image data is presented on a screen such as a display of a workstation, a computer, a tablet or a smartphone. Preferably, the device for eye tracking comprises a camera and is directed to the user for acquiring imaged of the user, in particular the eyes of the user.

In general, the visualized medical image data is divided into a plurality of regions, namely several first regions and/or second regions, i.e., each first region/second region forms an individual spatial region of the visualized medical image data set, and for each first region the control value is individually provided and respectively compared to the threshold value, in particular to the corresponding threshold value. Thereby, it is thinkable that all first regions have the same threshold value or alternatively that each first region has its own individualized threshold value. By assigning an individual threshold value, it is advantageously possible to force the user to focus on specified regions of the organ by weighting the threshold value correspondingly. The term "control value" represents a value that depends at least on the intensity of acquired eye orientations directed to the first region. For example, the control value is equal or proportional to the number and/or duration of acquired eye orientations directed to the first region, i.e. the control value is a representation of how often and/or how long (each time or in total) the user looked at a specific first region. Preferably, the user can only continue, when a defined portion of the visualized medical image data set is viewed.

According to a preferred embodiment of the present invention, the method further comprises:
- assigning the control value to the first region based on the intensity of the acquired eye orientations directed to the first region and an intensity of acquired eye orientations directed to a second region, in particular based on a difference between the intensity of the acquired eye orientations directed to the first region and eye orientations directed to the second region.

In other words, the control value depends on the difference of the intensities of respectively watching the first region and the second region. Thus, it is advantageously possible to make sure that the fist region is observed and/or viewed thoroughly enough compared to the second region. Thereby, the second region forms a spatial region of the visualized medical image data set which is separate from the first region. As a consequence, the visualized medical image data set is analysed homogeneously. For example, the first region and the second region both include relevant regions for a special finding / medical report. Thus, it is possible to avoid that the user accidently forgets to examine one of the two relevant regions.

In a preferred embodiment of the present invention, it is provided that the intensity of eye orientations comprises a number and/or a duration of acquired eye orientation directed to the first region or the second region. Thus, it may be taken into account that the user might look at a first region for a longer duration and/or more often. Since the eyes are usually constantly in motion, the number of times a user looked at a specific region (number of eye orientations directed to the region) may be a useful control value. As a consequence, the control value corresponds to an intensity of examining the first region.

In particular, the information is provided during the analysis, for example, after a defined time interval starting from the beginning of the analysis. Thus, the user can be sure that he observed and/or viewed each first region adequately and can finish the analysis, in particular after he views said first region. It is also thinkable that the user is informed when he wants to switch to another cross-sectional view and that the control value of one of the first regions is smaller than the respective threshold value. In some embodiments, the system is configured such that the user only can switch to the next cross sectional view when the control value exceeds the threshold value.

In particular, a control value is assigned to each first region being observed by the user. Thus, each first region being already observed can be marked for the subsequent analysis. For example, the first regions being already observed are coloured, wherein the colour is chosen according to a false colour scale related to the control value.

Preferably, the information is provided to the user or to another user after finishing the analysis. In particular, the user is only informed when the user is about to finish the analysis and one of the first regions has not been appropriately taken into account. As a consequence, the user is only informed when there is a problem and is not disturbed otherwise. It is also thinkable that the user is informed when an overall control value related to all first regions is smaller than an overall threshold value.

In other embodiments, another user who is about to perform a second analysis of the visualized medical image data set is informed which regions have been intensively viewed by the first user, of which regions are below the threshold, so he can direct his attention directly at these regions.

In particular, the first region is highlighted in the visualized medical image data set in order to provide the information. For example, the first region is highlighted by colour, a border, and/or an enlargement of the first region having a control value that is smaller than the threshold value. Thus, the user automatically looks at the first region and takes the highlighted first region into consideration. In particular, it is thinkable that the first regions having a control value greater than the threshold value are coloured in a first colour, such as blue, and that the first regions having a control value smaller than the threshold value are coloured in a second colour, such as red.

According to another embodiment, it is provided that the control value and/or the acquired eye orientation is saved, preferably together with the medical image data set and/or the visualized medical image data set. Thus, it is advantageously possible to focus a re-examination or a second analysis of the medical image data set on the first region having a low control value. Further, it is possible to provide the user a mention of the focus of the previous examination when he restarts the analysis/examination after saving and pausing the examination. Saving the control values and/or the acquired eye orientation might even be used as evidence for verifying that the medical image data set has been observed and/or viewed appropriately, for example, in a legal dispute.

In another embodiment of the present invention it is provided that for assigning eye orientations of the user to regions of the visualized medical image date set the first region and/or the second region is defined by a predefined pattern and/or by a specific part of an organ being identified. For example, the first and/or second regions are defined by a grid, wherein preferably a mesh size of the grid is adjustable. In this way, the first regions are set easily. For example, a two dimensional image which is part of the visualized medical image data set, may be divided into 8-30 time 8 -30 rectangular regions. By assigning the first regions to parts of organs, it is advantageously possible to adjust the respective threshold and thus to adapt the criteria for providing the information to the organ or the region of the organ, preferably automatically. In particular, it is possible to exclude parts of the visualized medical image data set that are irrelevant for the examination, such as a background region of the organ, and adapt the threshold values adequately. In other words, the threshold value for each first region is preferably adaptable based on the respective part of the organ visualized.

Preferably, an information about a region of interest is provided to the user when the control value is greater than the threshold value and/or a further threshold value. Thus, it is advantageously possible to inform another user about a region being of highest interest in a preceding examination. In particular, the first region which was region of interest is highlighted, for example by colouring, enlargement, and/or using a border.

According to the present invention, the method is supported by a machine learning mechanism and the machine learning mechanism, such as an artificial neuronal network, identifies an abnormality in the medical image data set and automatically adapts the threshold value for the first region having the abnormality. As a consequence, the user is directed to the first region that might otherwise be less important to him. According to the present invention it is also provided that the user gets additional information about the region of interest or the first region having a control value that does not exceed the threshold value. For example, the user is informed about interrelationships or problems that are known for the specific first region. As a consequence, the user can be asked to focus the examination on these first regions in more detail. In particular, an automatic landmarking and parsing of human anatomy (ALPHA) technology is used in addition to the classification of the first region by the respective control value. Preferably, it is provided that the machine learning mechanism is triggered, for example by a duration of the acquired eye-orientation directed to the first region, and subsequently depending on the first region a recommendation is provided to the user. An example for such a recommendation might be a massage such: "The region that you currently observe is known for having following problem and/or for being correlated to another region."

In particular, the threshold value is adjustable. Thus, the user can switch to a modus allowing a brief look at the visualized medical image data set without being disturbed by the information. In another modus, the threshold value is set for a detailed analysis.

Another aspect of the present invention is a system for supporting a visual analysis of a medical image data set, wherein the system is configured for
- providing a visualized medical image data set;
- performing an analysis of the visualized medical image data set by an user;
- acquiring an eye orientation of the user during analysis by a device for eye-tracking and
- assigning the eye orientations of the user to regions of the visualized medical image date set;
- assigning a control value to a first region based on an intensity of acquired eye orientations directed to the first region and
- providing an information to the user, when the control value is smaller and/or bigger than a threshold value for the first region.

A further aspect of the present invention is a computer program product for carrying out the steps of the method according to the present invention when the computer program product is loaded into a memory of a programmable device.

A further aspect of the present invention is a computer-readable medium on which program elements are stored that can be read and executed by a computer unit in order to perform steps of the method according to the present invention when the program elements are executed by the computer unit.

In the drawings:
- Fig. 1: schematically shows a system for supporting a visual analysis of a medical image data set according to a preferred embodiment of the present invention

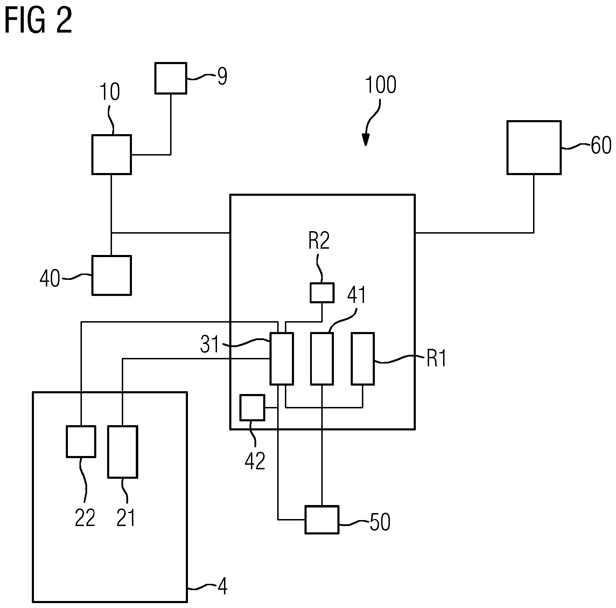

- Fig.2: schematically shows a block diagram illustrating a method for supporting a visual analysis of a medical image data set according to a preferred embodiment.
- Fig.3: schematically shows a flow diagram illustrating a method for supporting a visual analysis of a medical image data set according to a further preferred embodiment.

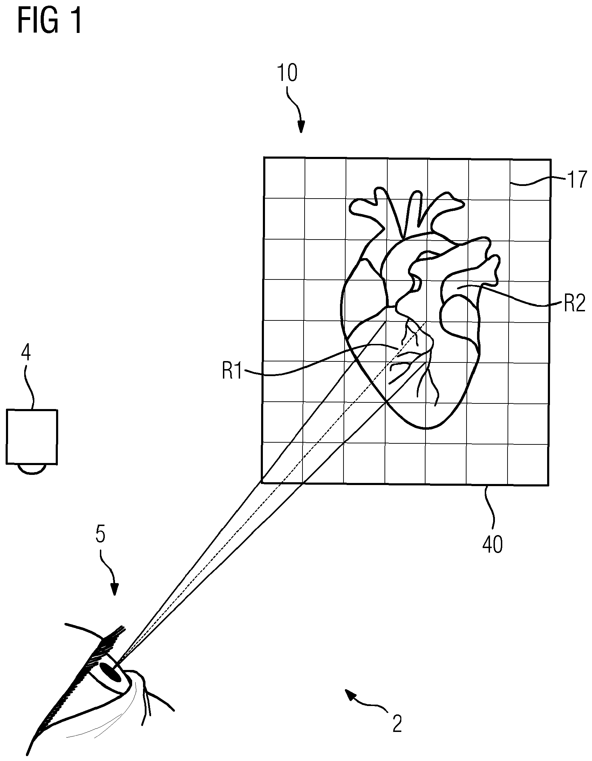

In Figure 1, a system 100 for supporting a visual analysis 2 of a visualized medical image data set 10 according to a preferred embodiment of the present invention is presented. Preferably, the system 1 is provided for visualizing a medical image data set 9, i.e., for reconstructing a medical image data set 9, for example, a medical image data set 9 recorded by a Computer Tomography (CT) device or a Magnetic Resonance Tomography (MRT) device. In general, the reconstructed medical image data set 9 is visualized on a screen 40, such as a display of a computer, a tablet, a smartphone, or the like, and consequently a user 5 can analyse a visualized medical image data 10 set being recorded by the CT device or the MRT device. Thereby, it is thinkable that the system 100 is configured for reconstructing the medical image data set 9. Alternatively, it is also thinkable that the reconstructed medical imaged data 9 set is provided to the system 100, for example, by transferring the reconstructed medical image data set. By visualizing the medical image data sets 9, it is possible to visually examine organs of a patient in detail. Preferably, the visualized medical image data set 10 is a sectional view of an organ or a three dimensional visualisation of the organ, such as a heart.

In general, the user 5 visually examines those parts of the visualized medical imaged data set 10 representing regions of interest to him, as illustrated in figure 1. For avoiding that the user 5 oversees an abnormality, an eye or a gaze tracking is provided. Thereby, a device 4 for eye or gaze tracking, such as a camera, is installed, for example, on or close to the screen 40. For example, the device 4 for eye or gaze tracking is mounted above or on top of the screen 40. By using the device 4 for eye or gaze tracking, it is advantageously possible to register an eye orientation of the user during analysis 2. In particular, it is provided that the eye orientations of the user are respectively assigned to regions of the visualized medical image date set. Thus, it is possible to assign a control value 31 to a first region R1, being one region of several regions of the visualized medical image data set. The control value 31 preferably represents the intensity and/or frequency of examination of the first region R1 by the user 5. In particular, the control value 31 is proportional to the intensity and/or frequency of the examination by the user 5. In particular, an information 50 is provided to the user 5 when the control value 31 is lower/bigger than a threshold value 41, in particular, a defined threshold value 41. Consequently, the user 5 is informed about those first regions R1 that have not been considered enough during the analysis 2. In particular, it is provided that the first region R1, having a control value 31 lower than the threshold value 41, is highlighted, for example by colouring or enlarging the first region R1. For instance, the first region R1 is coloured red when the control value 31 is smaller than the threshold value 41, and the first region R1 is coloured blue or green when the control value 31 is greater than the threshold value 41. It is also conceivable that the colouring continuously depends on the control value 31, i.e., the colour of the first regions R1 having a control value 31 lower than the threshold value 41 have different shades of red, in particular corresponding to the control value. The same might apply to the first regions having a control value greater than the threshold value. Thus, the user receives feedback about the intensity of examining the individual first region R1. It is also thinkable that the information 50 provided to the users comprises a communication message, such as a pop-up message or the like, that informs the user 5 by a phrase about the low control value 31 of the first region R1.
For homogenously examining a visualized medical image data set 10, the control value 31 is assigned to the first R region is based on the number of acquired eye orientations directed to the first region 21 and the number of eye orientations directed to a second region R2, in particular based on a difference between the number of the eye orientations 21 directed to the first region and the eye orientations 22 directed to the second region.

Preferably, the information 50 is provided after a set period of time after starting the analysis 2 by the user, in particular during the analysis 2, i.e., before finishing the analysis. Thus, the user 5 gets the information 50 during the analysis 2, and he is forced to examine the highlighted first region R1 before finishing the analysis 2.

Preferably, it is provided that the first regions R1 are defined by a pattern 17 or pixel by pixel. It is also thinkable that the system 100 automatically identifies the organ and/or parts of the organs for defining the first regions R1 and informs the user 5 directly about the need for re-examination of the first regions R1. It is also thinkable that the system automatically identifies first regions R1 as being irrelevant for the examination and rules those first regions R1 out. As a consequence, the user 5 is not informed about re-examination of regions being irrelevant. Preferably, a machine learning mechanism 60 supports identifying organs and/or parts of organs. For example, it is provided that the system 100 includes and/or communicates with a machine learning mechanism 60, such as an artificial neuronal network, for providing the information 50 to the user 5. Thus, the system 100 might inform the user 5 about the increased relevance of a specific first region R1. It is also thinkable that each first region R1 has its own threshold value 41, in particular, set by the machine learning mechanism 60. As a consequence it is advantageously possible to rate the different first regions 51 with respect to their relevance for the examination.

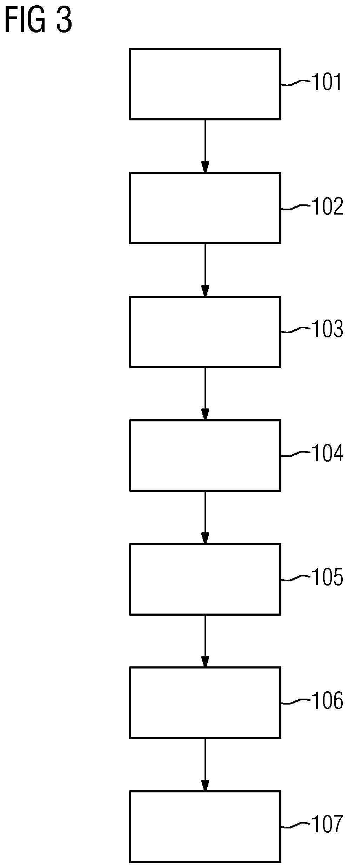

In figure 3, a flow chart illustrating a method for supporting a visual analysis 2 of a medical image data set 9 according to a further preferred embodiment is shown. In particular, the method includes:
- visualising 101 the medical image data set 9a and/or
- performing an analysis 101 of the visualized medical image data set by an user 5 and/or
- acquiring 102 an eye orientation of the user during analysis 2 and/or
- respectively assigning 103 the eye orientations 21 of the user 5 to regions of the visualized medical image date set 10 and/or
- assigning 104 a control value 31 to the first region R1 based on the number of eye orientations 21 directed to the first region and/or
- providing 105 an information 50 to the user 5 when the control value 31 is smaller than a threshold value 41 and/or
- highlighting 106 the first region R1 having a control value 31 smaller than the threshold value 41 and/or
- saving 107 the control value and/or the acquired eye orientation.

## Claims

1. Method for supporting a visual analysis (2) of a medical image data set (9), comprising:
- providing a visualized medical image data set (10);
- providing a device (16) for eye-tracking for observing an eye orientation of an user (5) during an analysis of the visualized medical image data set;
- assigning eye orientations of the user (5) to regions of the visualized medical image date set (10) for calibration;
- acquiring the eye orientation of the user (5) during analysis (2) of the visualized medical image data by the device (16) for eye-tracking
- assigning a control value (31) to a first region (R1) based on an intensity of acquired eye orientations (21) directed to the first region and
- providing an information (50) to the user (5) when the control value (31) is smaller and/or bigger than a threshold value (41) for the first region (R1) , wherein a machine learning mechanism (60), such as an artificial neuronal network, identifies an abnormality in the medical image data set (9) and automatically adapts the threshold value (41) for the first region (R1) having the abnormality and/or wherein the mechanism (60) provides additional information to the user about a region of interest when the control value (31) is bigger than the threshold value (41) and/or a further threshold value (42), or the first region (R1) having a control value (31) that does not exceed the threshold value (41).

2. Method according to claim 1, further comprising
- assigning the control value (31) to the first region (R1) based on the intensity of acquired eye orientations (21) directed to the first region and an intensity of acquired eye orientations (22) directed to a second region (R2), in particular based on a difference between the intensity of the acquired eye orientations directed to the first region (R1) and acquired eye orientations (22) directed to the second region (R2).

3. Method according to one of the preceding claims, wherein the intensity of acquired eye orientations (21) comprises a number and/or a duration of acquired eye orientations directed to the first region (R1) or the second region (R2) .

4. Method according to one of the preceding claims, wherein the information (50) is provided to the user during the analysis (2).

5. Method according to claim 4, wherein a control value (31) is assigned to each first region (R1) being observed by the user (5).

6. Method according to one of the preceding claims, wherein the information (50) is provided to the user or to another user after finishing the analysis (2).

7. Method according to one of the preceding claims, wherein the first region (R1) is highlighted in the visualized medical image data set (10) for providing the information (50) .

8. Method according to one of the preceding claims, wherein the control value (31) and/or the acquired eye orientation is saved, preferably together with the medical image data set (9) and/or the visualized medical image data set (10) .

9. Method according to one of the preceding claims, wherein an information about a region of interest is provided to the user, when the control value (31) is bigger than the threshold value and/or a further threshold value (42).

10. Method according to one of the preceding claims, wherein the threshold value (41) is adjustable.

11. System (100) for supporting a visual analysis of a medical image data set (9), wherein the system (100) is configured for
- providing a device (16) for eye-tracking for observing an eye orientation of an user (5) during an analysis of the visualized medical image data set;
- assigning eye orientations of the user (5) to regions of the visualized medical image date set (10) for calibration;
- the device (16) for eye-tracking acquiring the eye orientation of the user (5) during analysis (2) of the visualized medical image data;
- assigning a control value (31) to a first region (R1) based on an intensity of acquired eye orientations (21) directed to the first region and
- providing an information (50) to the user (5) when the control value (31) is smaller and/or bigger than a
threshold value (41) for the first region (R1), the system comprising a machine learning mechanism (60), such as an artificial neuronal network, configured to identify an abnormality in the medical image data set (9) and to automatically adapt the threshold value (41) for the first region (R1) having the abnormality and/or wherein the mechanism (60) is configured to provide additional information to the user about a region of interest when the control value (31) is bigger than the threshold value (41) and/or a further threshold value (42), or the first region (R1) having a control value (31) that does not exceed the threshold value (41).

12. Computer program product for carrying out the steps of the method according to one of the claims 1 to 10, when the computer program product is loaded into a memory of a programmable device.

13. Computer-readable medium on which program elements are stored that can be read and executed by a computer unit in order to perform steps of the method according to one of the claims 1 to 10, when the program elements are executed by the computer unit.

## Patentansprüche

1. Verfahren zur Unterstützung einer visuellen Analyse (2) eines medizinischen Bilddatensatzes (9), umfassend:
- Bereitstellen eines visualisierten medizinischen Bilddatensatzes (10);
- Bereitstellen einer Vorrichtung (16) für Augen-Tracking zum Beobachten einer Augenorientierung eines Benutzers (5) während einer Analyse des visualisierten medizinischen Bilddatensatzes;
- Zuordnen von Augenorientierungen des Benutzers (5) zu Regionen des visualisierten medizinischen Bilddatensatzes (10) zur Kalibrierung;
- Erfassen der Augenorientierung des Benutzers (5) während der Analyse (2) der visualisierten medizinischen Bilddaten durch die Vorrichtung (16) für Augen-Tracking;
- Zuordnen eines Kontrollwerts (31) zu einer ersten Region (R1) basierend auf einer Intensität von erfassten Augenorientierungen (21), die zur ersten Region gerichtet sind; und
- Bereitstellen einer Information (50) für den Benutzer (5), wenn der Kontrollwert (31) kleiner und/oder größer als ein Schwellenwert (41) für die erste Region (R1) ist, wobei ein Mechanismus für maschinelles Lernen (60), beispielsweise ein künstliches neuronales Netzwerk, eine Anomalie im medizinischen Bilddatensatz (9) identifiziert und den Schwellenwert (41) für die erste Region (R1) mit der Anomalie automatisch anpasst, und/oder wobei der Mechanismus (60) zusätzliche Informationen für den Benutzer über eine Region von Interesse bereitstellt, wenn der Kontrollwert (31) größer als der Schwellenwert (41) und/oder ein weiterer Schwellenwert (42) ist, oder die erste Region (R1) einen Kontrollwert (31) aufweist, der den Schwellenwert (41) nicht überschreitet.

2. Verfahren nach Anspruch 1, ferner umfassend:
- Zuordnen des Kontrollwerts (31) zur ersten Region (R1) basierend auf der Intensität von erfassten Augenorientierungen (21), die zur ersten Region gerichtet sind, und einer Intensität von erfassten Augenorientierungen (22), die zu einer zweiten Region (R2) gerichtet sind, insbesondere basierend auf einer Differenz zwischen der Intensität der erfassten Augenorientierungen, die zur ersten Region (R1) gerichtet sind, und der erfassten Augenorientierungen (22), die zur zweiten Region (R2) gerichtet sind.

3. Verfahren nach einem der vorhergehenden Ansprüche, wobei die Intensität von erfassten Augenorientierungen (21) eine Anzahl und/oder eine Dauer von erfassten Augenorientierungen umfasst, die zur ersten Region (R1) oder zur zweiten Region (R2) gerichtet sind.

4. Verfahren nach einem der vorhergehenden Ansprüche, wobei die Informationen (50) für den Benutzer während der Analyse (2) bereitgestellt werden.

5. Verfahren nach Anspruch 4, wobei jeder ersten Region (R1), die vom Benutzer (5) beobachtet wird, ein Kontrollwert (31) zugeordnet wird.

6. Verfahren nach einem der vorhergehenden Ansprüche, wobei die Informationen (50) für den Benutzer oder einen anderen Benutzer nach Beendigung der Analyse (2) bereitgestellt werden.

7. Verfahren nach einem der vorhergehenden Ansprüche, wobei die erste Region (R1) im visualisierten medizinischen Bilddatensatz (10) zum Bereitstellen der Informationen (50) hervorgehoben wird.

8. Verfahren nach einem der vorhergehenden Ansprüche, wobei der Kontrollwert (31) und/oder die erfasste Augenorientierung vorzugsweise zusammen mit dem medizinischen Bilddatensatz (9) und/oder dem visualisierten medizinischen Bilddatensatz (10) gespeichert werden.

9. Verfahren nach einem der vorhergehenden Ansprüche, wobei eine Information über eine Region von Interesse für den Benutzer bereitgestellt wird, wenn der Kontrollwert (31) größer als der Schwellenwert und/oder ein weiterer Schwellenwert (42) ist.

10. Verfahren nach einem der vorhergehenden Ansprüche, wobei der Schwellenwert (41) einstellbar ist.

11. System (100) zum Unterstützen einer visuellen Analyse eines medizinischen Bilddatensatzes (9), wobei das System (100) konfiguriert ist zum:
- Bereitstellen einer Vorrichtung (16) für Augen-Tracking zum Beobachten einer Augenorientierung eines Benutzers (5) während einer Analyse des visualisierten medizinischen Bilddatensatzes;
- Zuordnen von Augenorientierungen des Benutzers (5) zu Regionen des visualisierten medizinischen Bilddatensatzes (10) zur Kalibrierung;
- Erfassen der Augenorientierung des Benutzers (5) während der Analyse (2) des visualisierten medizinischen Bilddatensatzes durch die Vorrichtung (16) für Augen-Tracking;
- Zuordnen eines Kontrollwerts (31) zu einer ersten Region (R1) basierend auf einer Intensität von erfassten Augenorientierungen (21), die zur ersten Region gerichtet sind, und
- Bereitstellen einer Information (50) für den Benutzer (5), wenn der Kontrollwert (31) kleiner und/oder größer als ein Schwellenwert (41) für die erste Region (R1) ist, wobei das System einen Mechanismus für maschinelles Lernen (60), beispielsweise ein künstliches neuronales Netzwerk, umfasst, der so konfiguriert ist, dass er eine Anomalie im medizinischen Bilddatensatz (9) identifiziert und den Schwellenwert (41) für die erste Region (R1) mit der Anomalie automatisch anpasst, und/oder wobei der Mechanismus (60) so konfiguriert ist, dass er zusätzliche Informationen für den Benutzer über eine Region von Interesse bereitstellt, wenn der Kontrollwert (31) größer als der Schwellenwert (41) und/oder ein weiterer Schwellenwert (42) ist, oder die erste Region (R1) einen Kontrollwert (31) aufweist, der den Schwellenwert (41) nicht überschreitet.

12. Computerprogrammprodukt zum Ausführen der Schritte des Verfahrens nach einem der Ansprüche 1 bis 10, wenn das Computerprogrammprodukt in einen Speicher einer programmierbaren Vorrichtung geladen ist.

13. Computerlesbares Medium, auf welchem Programmelemente gespeichert sind, die von einer Computereinheit ausgelesen und ausgeführt werden können, um die Schritte des Verfahrens nach einem der die Ansprüche 1 bis 10 auszuführen, wenn die Programmelemente von der Computereinheit ausgeführt werden.

## Revendications

1. Procédé pour venir à l'appui d'une analyse (2) visuelle d'un ensemble (9) de données d'image médicale, comprenant :
- on se procure un ensemble (10) de données d'image médicale visualisées ;
- on se procure un dispositif (16) de suivi d'œil pour observer une orientation de l'œil d'un utilisateur (5) pendant une analyse de l'ensemble de données d'image médicale visualisées ;
- on affecte des orientations de l'œil de l'utilisateur (5) à des régions du jeu (10) de données d'image médicale visualisées pour étalonnage ;
- on acquiert l'orientation de l'œil de l'utilisateur (5) pendant une analyse (2) de la donnée d'image médicale visualisée par le dispositif (16) pour le suivi d'œil,
- on affecte une valeur (31) de commande à une première région (R1) sur la base d'une intensité de l'orientation (21) de l'œil acquise dirigée sur la première région et
- on fournit une information (50) à l'utilisateur (5), lorsque la valeur (31) de commande est plus petite et/ou plus grande qu'une valeur (41) de seuil pour la première région (R1),
dans lequel un mécanisme (60) d'apprentissage automatique,
tel qu'un réseau neuronal artificiel, identifie une anomalie dans le jeu (9) de données d'image médicale et adapte automatiquement la valeur (41) de seuil pour la première région (R1) ayant l'anomalie et/ou dans lequel le mécanisme (60) fournit une information supplémentaire à l'utilisateur sur une région à laquelle on s'intéresse, lorsque la valeur (31) de commande est plus grande que la valeur (41) de seuil et/ou une autre valeur (42) de seuil,
ou la première région (R1) ayant une valeur (31) de commande, qui n'excède pas la valeur (41) de seuil.

2. Procédé suivant la revendication 1, comprenant, en outre
- on affecte la valeur (31) de commande à la première région (R1) sur la base de l'intensité d'orientations (21) acquises de l'œil dirigées sur la première région, et d'une intensité d'orientations (22) acquises de l'œil dirigées sur une deuxième région (R2), reposant en particulier sur une différence entre l'intensité d'orientations acquises de l'œil dirigées sur la première région (R1) et d'orientations (22) acquises de l'œil dirigées sur la deuxième région (R2).

3. Procédé suivant l'une des revendications précédentes, dans lequel l'intensité d'orientations (21) acquises de l'œil comprend un nombre et/ou une durée d'orientations acquise de l'œil dirigées sur la première région (R1) ou sur la deuxième région (R2).

4. Procédé suivant l'une des revendications précédentes, dans lequel l'information (50) est fournie à l'utilisateur pendant l'analyse (2).

5. Procédé suivant la revendication 4, dans lequel on affecte une valeur (31) de commande à chaque première région (R1) observée par l'utilisateur (5).

6. Procédé suivant l'une des revendications précédentes, dans lequel on fournit l'information (50) à l'utilisateur ou à un autre utilisateur après avoir fini l'analyse (2).

7. Procédé suivant l'une des revendications précédentes, dans lequel on met en exergue la première région (R1) dans l'ensemble (10) de données d'image médicale visualisées pour fournir l'information (50).

8. Procédé suivant l'une des revendications précédentes, dans lequel on sauvegarde la valeur (31) de commande et/ou l'orientation acquise de l'œil, de préférence ensemble avec l'ensemble (9) de données d'image médicale et/ou l'ensemble (10) de données d'image médical visualisées.

9. Procédé suivant l'une quelconque des revendications précédentes, dans lequel on fournit à l'utilisateur une information sur la région à laquelle on s'intéresse, lorsque la valeur (31) de commande est plus grande que la valeur de seuil et/ou qu'une autre valeur (42) de seuil.

10. Procédé suivant l'une quelconque des revendications précédentes, dans lequel la valeur (41) de seuil est réglable.

11. Système (100) pour venir à l'appui d'une analyse visuelle d'un ensemble (9) de données d'image médicale, le système (100) étant configuré pour
- procurer un dispositif (16) de suivi d'œil pour observer une orientation de l'œil d'un utilisateur (5) pendant une analyse de l'ensemble de données d'image médicale visualisées ;
- affecter des orientations de l'œil de l'utilisateur (5) à des régions de l'ensemble (10) de données d'image médicale visualisées pour étalonnage ;
- le dispositif (16) de suivi de l'œil acquérant l'orientation de l'œil de l'utilisateur (5) pendant une analyse (2) de la donnée d'image médicale visualisée ;
- affecter une valeur (31) de commande à une première région (R1) sur la base d'une intensité d'orientations (21) acquises de l'œil dirigées sur la première région et
- fournir une information (50) à l'utilisateur (5), lorsque la valeur (31) de commande est plus petite et/ou plus grande qu'une valeur (41) de seuil pour la première région (R1),
le système comprenant un mécanisme (60) d'apprentissage automatique, tel qu'un réseau neuronal artificiel, configuré pour identifier une anomalie dans l'ensemble (9) de données d'image médicale et pour adapter automatiquement la valeur (41) de seuil pour la première région (R1) ayant l'anomalie et/ou dans lequel le mécanisme (60) est configuré pour fournir une information supplémentaire à l'utilisateur sur une région à laquelle on s'intéresse, lorsque la valeur (31) de commande est plus grande que la valeur (41) de seuil et/ou une autre valeur (42) de seuil, ou la première région (R1) ayant une valeur (31) de commande, qui n'excède pas la valeur (41) de seuil.

12. Produit de programme d'ordinateur pour effectuer les stades du procédé suivant l'une des revendications 1 à 10, lorsque le produit de programme d'ordinateur est chargé dans une mémoire d'un dispositif programmable.

13. Support déchiffrable par ordinateur, sur lequel les éléments de programme sont mis en mémoire, qui peuvent être lus et exécutés par une unité informatique pour effectuer des stades du procédé suivant l'une des revendications 1 à 10, lorsque les éléments de programme sont exécutés par l'unité informatique.
